# EUROPÄISCHE PATENTSCHRIFT

(11) **EP 2 736 430 B1**
(45) Veröffentlichungstag und Bekanntmachung des Hinweises auf die Patenterteilung: **02.03.2016**
(21) Anmeldenummer: 12761943.5
(22) Anmeldetag: 14.09.2012
(51) Int. Cl.: A61B 17/70, A61B 17/80

(54) **IMPLANTIERBARE VORRICHTUNG**
IMPLANTABLE DEVICE
DISPOSITIF POUVANT ÊTRE IMPLANTÉ

(30) Priorität: 14.09.2011 EP 11007513
(43) Veröffentlichungstag der Anmeldung: 04.06.2014
(73) Patentinhaber: Zimmer GmbH, 8404 Winterthur (CH)
(72) Erfinder: KUSTER, Markus, Mosman Park, Western Australia 6012 (AU)
(74) Vertreter: Manitz, Finsterwald & Partner GbR
(86) Internationale Anmeldenummer: PCT/EP2012/068065
(87) Internationale Veröffentlichungsnummer: WO 2013/037939

(56) Entgegenhaltungen:
- WO-A1-99/09903
- WO-A1-2008/119006
- US-A- 6 022 350
- US-A1- 2004 087 951

## Beschreibung

Die vorliegende Erfindung betrifft eine implantierbare Vorrichtung. Beispielsweise wird zur Behandlung einer Knochenfraktur die Fraktur nach Ausrichtung der einzelnen Knochenfragmente in ihre korrekte Lage (Reposition) mittels Stiften, Schrauben, Drähten oder Platten fixiert. Insbesondere kann zur Fixierung eine Platte eingesetzt werden, die mittels Verankerungselementen, insbesondere Knochenschrauben, an den einzelnen Knochenfragmenten befestigt ist. Bei dieser Art der Behandlung wird eine Kompressionswirkung erzielt, derart, dass die einzelnen Knochenfragmente durch die Platte und die Schrauben in einer festen Position zueinander gehalten und aneinander gedrückt werden. Die implantierbare Vorrichtung kann aber z.B. auch zur Versteifung eines Wirbelsäulensegments, insbesondere zur Befestigung einer Platte zwischen zwei Wirbeln, oder zur Befestigung eines Gelenkersatzes wie eines Hüftimplantats oder eines Schulterimplantats verwendet werden.

Das Dokument US 5,904,683 A beschreibt eine implantierbare Vorrichtung mit Verschlusselementen, um polyaxiale Knochenschrauben winkelstabil in einer Platte zu verblocken. Das Dokument EP 0 828 459 B1 zeigt ebenfalls eine implantierbare Vorrichtung, bei der zusätzlich ein ringartiger Einsatz vorgesehen ist, um die jeweilige polyaxiale Knochenschraube raumfest in einer Platte zu fixieren. Das Dokument US 2004/0087951 A1 offenbart eine implantierbare Vorrichtung gemäß den Merkmalen des Oberbegriffs des Anspruchs 1.

Die implantierbare Vorrichtung umfasst ein Implantat, das wenigstens eine Öffnung aufweist. Die implantierbare Vorrichtung ist insbesondere eine Knochenfixierungsvorrichtung, insbesondere zur operativen Versorgung von Frakturen. Das Implantat ist insbesondere ein Fixierungselement, insbesondere eine Frakturfixierungsplatte.

Die implantierbare Vorrichtung umfasst ferner wenigstens ein Verankerungselement zur Befestigung des Implantats an zumindest einem Knochen. Das jeweilige Verankerungselement umfasst einen Schaft und einen Kopf, wobei wenigstens ein Teil des Schafts durch die jeweilige Öffnung hindurchführbar ist, während der Kopf in der jeweiligen Öffnung lagerbar ist.

Die implantierbare Vorrichtung weist zudem einen Arretiermechanismus mit einem Feststellelement und einem radial verformbaren Ringelement auf, das durch Betätigung des Feststellelements gegen den Kopf des Verankerungselement pressbar ist, um diesen in der Öffnung des Implantats zu fixieren.

Die Öffnung des Implantats weist eine Vertiefung auf, die eine Auflagefläche für den Kopf des Verankerungselements aufweist, insbesondere derart, dass in einem zusammengesetzten Zustand der implantierbaren Vorrichtung der Kopf an der Auflagefläche anliegt. Insbesondere wirken beim Zusammensetzen der implantierbaren Vorrichtung ausschließlich das Ringelement und das Feststellelement derart unmittelbar zusammen, dass das Ringelement radial komprimiert wird. Die Vertiefung ist an einer proximalen Seite des Implantats ausgebildet. Insbesondere handelt es sich bei der Vertiefung um eine Versenkung. Unter der proximalen Seite ist die während der Operation dem Operateur zugewandte Seite des Implantats zu verstehen, unter einer distalen Seite die vom Operateur abgewandte Seite.

Durch die Integration des Arretiermechanismus in das Implantat kann eine niedrige Bauhöhe des Implantats erreicht werden. Durch die zusätzliche Verwendung des Ringelements können das Verankerungselement und das Feststellelement hinsichtlich der Drehbewegung beim Festziehen des Feststellelements zumindest teilweise voneinander entkoppelt werden. Da der Kopf unmittelbar, insbesondere nicht mittelbar über das Ringelement, auf dem Implantat aufliegt, kann ein Drehen der Auflagefläche relativ zu dem Kopf beim Festziehen des Feststellelements verhindert werden. Dies ermöglicht eine zuverlässige Verblockung des oder der Verankerungselemente in dem Implantat.

In einer Ausführungsform sind das Implantat und der Kopf des Verankerungselements zur polyaxialen Lagerung des Verankerungselements in der jeweiligen Öffnung des Implantats ausgebildet. Insbesondere ist die Verankerungsvorrichtung derart ausgebildet, dass vor Fixierung des Kopfs in der Öffnung eine Winkelstellung des Verankerungselements relativ zu einer Achse der Öffnung bzw. dem Implantat innerhalb eines vorgegebenen Bereichs frei einstellbar ist.

In einer weiteren Ausführungsform ist das Ringelement in einem zusammengesetzten Zustand der implantierbaren Vorrichtung zwischen dem Feststellelement und dem Kopf des Verankerungselements angeordnet. Das Ringelement kann in einem monierten Zustand der implantierbaren Vorrichtung um einen ballig ausgeführten Abschnitt des Kopfs des Verankerungselements herum angeordnet sein. Insbesondere ist das Ringelement und/oder das Feststellelement radial von und/oder konzentrisch zu dem Kopf angeordnet.

In einer weiteren Ausführungsform ist das Ringelement ein geschlitzter Ring. Alternativ oder zusätzlich kann das Ringelement radial elastisch komprimierbar und/oder zusammendrückbar sein, insbesondere durch das Feststellelement. Durch das Feststellelement kann das Ringelement mit einer radialen Kraft beaufschlagt werden.

In einer beispielhaften Ausführungsform ist eine radial äußere Geometrie des Ringelements zumindest abschnittsweise konusförmig ausgebildet, wobei ein Öffnungswinkel des konusförmigen Abschnitts insbesondere 1° bis 12°, insbesondere 2° bis 8° beträgt. Eine konusförmige Ausgestaltung lässt sich durch die Geometrie eines Mantels eines Kegelstumpfs beschreiben. Unter dem Öffnungswinkel ist der doppelte Wert eines Winkels zwischen einer Mantellinie des Kegelstumpfs und seiner Rotationsachse zu verstehen.

In einer weiteren Ausführungsform ist eine radial innere Geometrie des Feststellelements zumindest abschnittsweise konusförmig ausgebildet, wobei ein Öffnungswinkel des konusförmigen Abschnitts 1° bis 12°, insbesondere 2° bis 8° beträgt. Insbesondere ist die Innengeometrie des Feststellelements komplementär zur Außengeometrie des Ringelements ausgebildet.

Dabei ist es ausreichend, wenn wenigstens eine der beiden Geometrien, d.h. entweder die radial äußere Geometrie des Ringelements oder die radial innere Geometrie des Feststellelements zumindest abschnittsweise konusförmig ausgebildet ist. Bevorzugt sind beide Geometrien zumindest abschnittsweise konusförmig ausgebildet. Insbesondere sind die beiden konusförmigen Abschnitte komplementär zueinander ausgebildet.

In einer Ausführungsform verjüngt sich der jeweilige konusförmige Abschnitt des Ringelements und/oder des Feststellelements entgegen einer Einführrichtung des Verankerungselements. Das Verankerungselement wird von proximal nach distal in das Implantat bzw. die darin ausgebildete Öffnung eingeführt. Demnach weist die Einführrichtung von der proximalen Seite des Implantats zu der distalen Seite des Implantats. Im implantierten Zustand der implantierbaren Vorrichtung weist der Kopf des Verankerungselements in proximale Richtung, eine Spitze des Verankerungselements in distale Richtung.

In einer weiteren Ausführungsform ist eine mit dem Kopf des Verankerungselements zusammenwirkende radiale Innenseite des Ringelements und/oder zumindest ein mit dem Ringelement zusammenwirkender Abschnitt des Kopfs zur Erhöhung einer zwischen dem Kopf und dem Ringelement wirkenden Reibung oberflächenbehandelt, insbesondere aufgerauht.

In einer weiteren Ausführungsform ist das Feststellelement in die Öffnung des Implantats, insbesondere in die Vertiefung der Öffnung des Implantats, einschraubbar.

Insbesondere ist das Verankerungselement eine polyaxiale Schraube. In einer weiteren Ausführungsform weist die Vertiefung der Öffnung eine konkave Auflagefläche für den Kopf des Verankerungselements auf. Alternativ oder zusätzlich ist der Kopf des Verankerungselements an dem dem Schaft zugewandten Ende ballig ausgeführt, derart, dass das Verankerungselement vor einer Fixierung hinsichtlich seiner Winkellage relativ zu dem Implantat variabel positionierbar ist.

In einer Ausführungsform ist der komplette Schaft des Verankerungselements, jedoch nicht der Kopf des Verankerungselements, durch die Öffnung des Implantats hindurchführbar, wobei insbesondere die Dimensionen des Kopfs und der Öffnung derart aufeinander abgestimmt sind, dass der Kopf nicht durch die Öffnung hindurchführbar ist.

In einer weiteren Ausführungsform ist das Feststellelement eine Ringmutter.

In einer Ausführungsform ist bzw. sind eine oder mehrere Schrägen, die beim Zusammensetzen der implantierbaren Vorrichtung derart wirkt bzw. wirken, insbesondere zusammenwirken, dass das Ringelement radial komprimiert wird, ausschließlich an dem Ringelement und/oder dem Feststellelement ausgebildet.

Die verschiedenen angegebenen Ausführungsformen einer implantierbaren Vorrichtung gemäß dem auf die implantierbare Vorrichtung gerichteten unabhängigen Patentanspruch bzw. die dort realisierten Merkmale können selbstverständlich untereinander kombiniert werden. Nachfolgend wird die Erfindung anhand von den in den Zeichnungen illustrierten Ausführungsbeispielen näher erläutert. Dabei sollen die Ausführungsbeispiele und die Zeichnungen nur instruktiv verstanden werden und sollen nicht zur Einschränkung der in den Ansprüchen beschriebenen Gegenstände dienen. Die Darstellungen in den Zeichnungen sind vereinfacht; für das Verständnis der Erfindung nicht notwendige Einzelheiten sind weggelassen werden. Die Zeichnungen zeigen:
- Fig. 1: eine Draufsicht auf einen Abschnitt einer Knochenfixierungsvorrichtung gemäß einer Ausführungsform, und
- Fig. 2 und 3: einen Querschnitt durch die in Fig. 1 gezeigte Ausführungsform in verschiedenen Zuständen.

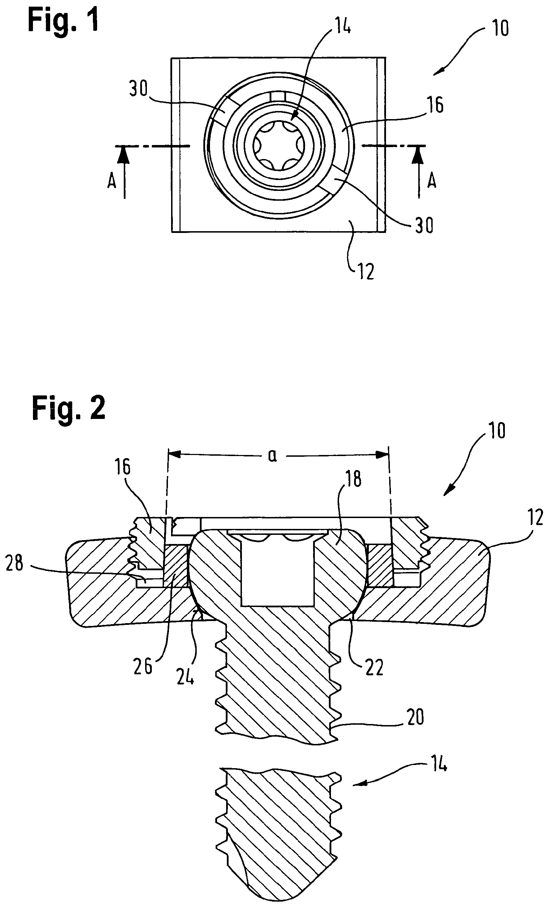

Fig. 1 zeigt eine Knochenfixierungsvorrichtung 10 mit einer länglichen, aber lediglich ausschnittsweise dargestellten Frakturfixierungsplatte 12 und einer Knochenschraube 14, die durch eine Öffnung 22 der Platte 12 hindurchgeführt ist. Neben der gezeigten Öffnung 22 weist die Platte 12 wenigstens eine weitere Öffnung auf, durch die jeweils eine weitere Knochenschraube hindurchführbar ist. Weitere Öffnungen und/oder Knochenschrauben können analog zu der gezeigten Öffnung 22 bzw. Knochenschraube 14 ausgebildet sein.

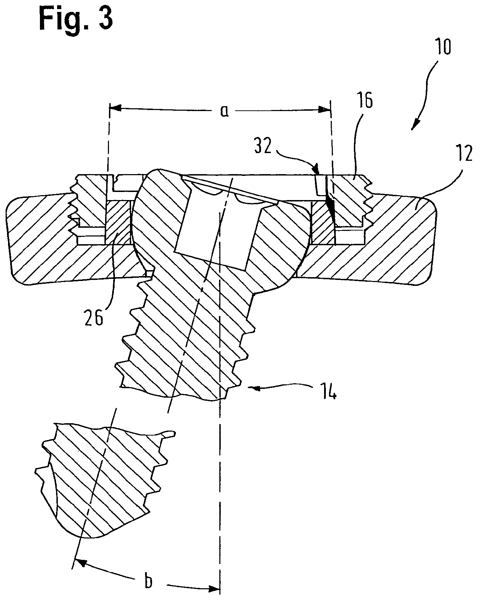

Die Knochenschraube 14 kann mittels eines geeigneten Werkzeugs an einem Knochen eines Patienten befestigt werden, um die Platte 12 an diesem zu fixieren. Bei der Knochenschraube 14 handelt es sich um eine so genannte polyaxiale Schraube, die innerhalb eines vorgegebenen Winkelbereichs in der geeigneten Winkelstellung relativ zu der Platte 12 zuverlässig mit dieser verbunden werden kann. Zu einer winkelstabilen Verblockung der Schraube 14 in der Platte 12 ist ein Arretiermechanismus mit einer Ringmutter 16 vorgesehen. Für weitere Öffnungen und/oder Knochenschrauben können analoge Arretiermechanismen vorgesehen sein. Zur Verdeutlichung der Funktionsweise des Arretiermechanismus zur Verblockung der Schraube 14 an der Platte 12 ist in den Fig. 2 und 3 jeweils ein Querschnitt durch die Knochenfixierungsvorrichtung 10 entlang der Schnittebene A-A der Fig. 1 dargestellt.

Anhand von Fig. 2 ist zu erkennen, dass die Schraube 14 einen ballig ausgeformten Kopf 18 und einen mit einem Außengewinde versehenen Schaft 20 aufweist. Zur Befestigung der Platte 12 an einem Knochen wird die Schraube 14 durch die Öffnung 22 der Platte 12 gesteckt und in den Knochen geschraubt, bis der Kopf 18 an einer konkaven Auflagefläche 24 der Öffnung 22 anliegt. Durch die ballige Ausgestaltung des Kopfs 18 und der entsprechenden Formgebung der Auflagefläche 24 kann die Knochenschraube 14 in verschiedenen Winkeln relativ zu der Platte 12 an dieser befestigt werden. Zu diesem Zeitpunkt ist die Schraube 14 bereits zuverlässig in dem Knochen verankert und erst anschließend und unabhängig davon erfolgt das Verblocken der Schraube 14 mit der Platte 12 mit Hilfe des Arretiermechanismus.

Der Arretiermechanismus umfasst die vorstehend bereits erwähnte Ringmutter 16 und einen Ring 26. Nachdem die Knochenschraube 14 in den Knochen geschraubt wurde, liegt der Kopf 18 an der Auflagefläche 24 der Öffnung 22. Der Ring 26, der in eine Vertiefung 28 der Öffnung 22 ragt, liegt an dem noch freiliegenden Teil des Kopfs 18 an. Der Ring 26 ist insbesondere ein geschlitzter Ring, der in einem entspannten Zustand etwas Spiel in Bezug auf den Kopf 18 hat oder lediglich leicht an diesem anliegt, derart, dass der Kopf 18 der Knochenschraube 14 noch bewegt, insbesondere verdreht bzw. verschwenkt werden kann. Die Vertiefung 28 weist ein Innengewinde auf, so dass die mit einem Außengewinde versehene Ringmutter 16 in die Vertiefung 28 der Öffnung 22 eingeschraubt werden kann. Durch das Einschrauben der Ringmutter 16 wird der Ring 26 radial komprimiert, was dadurch erreicht wird, dass der Ring 26 eine konusförmige Außengeometrie und/oder die Ringmutter 16 eine konusförmige Innengeometrie hat. Mit anderen Worten entspricht seine radiale Außenfläche im Wesentlichen einer Mantelfläche eines Kegelstumpfs, die sich in einer Richtung von dem Knochen weg verjüngt. Ein Öffnungswinkel a des Kegelstumpfs beträgt beispielsweise 5°, wobei unter dem Öffnungswinkel der doppelte Winkel zwischen einer Rotationsachse des Kegelstumpfs und einer Mantellinie zu verstehen ist. Die Ringmutter 16 weist im Ausführungsbeispiel eine komplementäre Innengeometrie auf, die es ermöglicht, die Ringmutter 16 ein Stück weit über die konische Außenseite des Rings 26 zu führen und die es andererseits gewährleistet, dass bei einem axialen Übereinanderschieben die Innenseite der Ringmutter 16 mit der Außenseite des Rings 26 in Kontakt kommt. Beim Eindrehen der Ringmutter 16 wird der Ring 26 insbesondere in radialer Richtung komprimiert, so dass die Innenseite des Rings 26 gegen den Kopf 18 der Schraube 14 gepresst wird. Die Verformung des Rings 26 ist elastisch, so dass der Ring 26 bei Entfernen der Ringmutter 16 im Wesentlichen wieder seine ursprüngliche Form annimmt. Dies kann auch durch die Wahl eines geeigneten Materials für den Ring 26 erreicht werden. In bestimmten Fällen kann eine - eventuell auch nur teilweise - plastische Verformung des Rings 26 vorgesehen sein.

Letztlich wird der Kopf 18 der Schraube 14 über den Ring 26 von der Ringmutter 16 mit einer Kraft beaufschlagt, um die Schraube 14 relativ zu der Platte 12 winkelstabil zu fixieren. Diese Kraft wird durch das Eindrehen der Ringmutter 16 bewirkt. Um das Eindrehen zu erleichtern, weist die Ringmutter 16 Werkzeugansätze auf, beispielsweise zwei radial verlaufende Schlitze 30, die in Fig. 1 gezeigt sind.

Das Funktionsprinzip des Arretiermechanismus beruht somit im Wesentlichen darauf, eine axial wirkende Kraft, die durch ein Eindrehen der Ringmutter 16 erzeugt wird, durch eine geeignete Ausgestaltung der Innengeometrie der Ringmutter 16 und der Außengeometrie des Rings 26 in eine radial nach innen auf den Kopf 18 wirkende Kraft umzuwandeln. Um die Reibung zwischen dem Ring 26 und dem mit dessen Innenseite zusammenwirkenden Abschnitt des Kopfs 18 zu erhöhen, sind die Innenseite des Rings und/oder der Schraubenkopf aufgerauht.

Fig. 3 zeigt die Knochenfixierungsvorrichtung 10 in einer etwas anderen Situation als in Fig. 2. Die Knochenschraube 14 erstreckt sich nicht im Wesentlichen senkrecht zu der Platte 12, sondern ist um einen Winkel b verkippt. Trotzdem ermöglicht der vorstehend ausführlich beschriebene Arretiermechanismus eine zuverlässige raumfeste Fixierung der Schraube 14 an der Platte 12.

Grundsätzlich muss nicht die gesamte Innengeometrie des Rings 16 bzw. die gesamte Außengeometrie des Rings 26 der Mantelfläche einem einzigen Konus entsprechen. In anderen Ausführungsformen ist unter anderem beispielsweise auch eine Kombination einer Zylindermantelfläche mit einem Konus oder die Kombination zweier Kegelstümpfe mit unterschiedlichen Öffnungswinkeln denkbar.

Den beschriebenen Ausführungsformen ist gemeinsam, dass die axiale Bewegung der Ringmutter 16 in eine, insbesondere elastische Komprimierung des Rings 26 umgewandelt wird, der dadurch gegen den Kopf 18 gepresst wird. Dabei passt sich die Innengeometrie des Rings 26 zumindest teilweise an die Formgebung des Kopfs 18, um eine möglichst große Wirkfläche zwischen dem Ring 26 und dem Kopf 18 zu erzeugen. So wird der Kopf 18 zuverlässig mit dem Ring 26 verbunden, der wiederum durch die Ringmutter 16 gesichert ist.

Die Knochenfixierungsvorrichtung 10 kann vormontiert werden. Hierzu wird der Ring 26 und die Ringmutter 16 bereits vor der Befestigung der Frakturfixierungsplatte 12 an einem Knochen bzw. bereits vor dem Einführen der Knochenschraube 14 in die Vertiefung 28 der Frakturfixierungsplatte 12 eingesetzt. Der Schaft 20 und der Kopf 18 des Verankerungselements 14 sind durch eine Öffnung des ringförmigen Feststellelements 16 hindurchführbar. Der Schraubenkopf 18 kann dann - insbesondere unter radialer Erweiterung des Rings 26 - in diesen hineingedrückt werden. Um ein Herausfallen des Rings 26 aus der vormontierten Knochenfixierungsvorrichtung 10 zu verhindern, ist eine entsprechende Sicherung 32 vorgesehen.

Die implantierbare Vorrichtung kann auch in anderen Bereichen als zur operativen Versorgung von Frakturen eingesetzt werden. Beispielsweise kann das Implantat auch als Hüftschale oder Wirbelsäulenplatte ausgebildet sein.

Im Lichte der hier gemachten Ausführungen eröffnen sich dem Fachmann weitere Ausführungsformen der in den Ansprüchen gekennzeichneten Erfindung, welche hier nicht abschließend dargestellt werden können. Alle sowohl in den Ansprüchen als auch in der Beschreibung und in den Zeichnungen in Verbindung mit der implantierbaren Vorrichtung gegebenenfalls verwendeten und gemäß der fachüblichen Konvention gewählten Ausrichtungs-, Positionierungs-, Orientierungs- und Richtungsangaben, die insbesondere anatomische Achsen, Ebenen, Richtungen im Raum und Bewegungsrichtungen betreffen, sind dem Fachmann bekannt und beziehen sich auf den implantierten Zustand der implantierbaren Vorrichtung.

### Bezugszeichenliste

- 10: Knochenfixierungsvorrichtung
- 12: Frakturfixierungsplatte
- 14: Knochenschraube
- 16: Ringmutter
- 18: Kopf
- 20: Schaft
- 22: Öffnung
- 24: Auflagefläche
- 26: Ring
- 28: Vertiefung
- 30: Schlitz
- 32: Sicherung
- A-A: Schnittebene
- a: Öffnungswinkel
- b: Winkel

## Patentansprüche

1. Implantierbare Vorrichtung, umfassend
- ein Implantat (12), wobei das Implantat (12) wenigstens eine Öffnung (22) aufweist,
- wenigstens ein Verankerungselement (14) zur Befestigung des Implantats (12) an zumindest einem Knochen, wobei das jeweilige Verankerungselement (14) einen Schaft (20) und einen Kopf (18) umfasst, und wobei wenigstens ein Teil des Schafts (20) durch die jeweilige Öffnung (22) hindurchführbar ist, während der Kopf (18) in der jeweiligen Öffnung (22) lagerbar ist, und
- einen Arretiermechanismus mit einem radial verformbaren Ringelement (26), wobei die Öffnung (22) des Implantats (12) eine Vertiefung (28) aufweist, die eine Auflagefläche (24) für den Kopf (22) des Verankerungselements (14) aufweist,
**dadurch gekennzeichnet, dass**
der Arretiermechanismus ein Feststellelement (16) umfasst, wobei das Ringelement (26) durch Betätigung des Feststellelements (16) gegen den Kopf (18) des Verankerungselement (14) pressbar ist, um diesen in der Öffnung (22) des Implantats (12) zu fixieren, und wobei beim Zusammensetzen der implantierbaren Vorrichtung ausschließlich das Ringelement (26) und das Feststellelement (16) derart unmittelbar zusammenwirken, dass das Ringelement (26) radial komprimiert wird.

2. Implantierbare Vorrichtung nach Anspruch 1,
**dadurch gekennzeichnet, dass**
das Implantat (12) und der Kopf (18) des Verankerungselements (14) zur polyaxialen Lagerung des Verankerungselements (14) in der jeweiligen Öffnung (22) des Implantats (12) ausgebildet sind.

3. Implantierbare Vorrichtung nach Anspruch 1 oder 2,
**dadurch gekennzeichnet, dass**
das Ringelement (26) in einem zusammengesetzten Zustand der Implantierbare Vorrichtung zwischen dem Feststellelement (16) und dem Kopf (18) des Verankerungselements (14) und/oder um einen ballig ausgeführten Abschnitt des Kopfs (18) des Verankerungselements (14) herum angeordnet ist.

4. Implantierbare Vorrichtung nach zumindest einem der vorstehenden Ansprüche,
**dadurch gekennzeichnet, dass**
das Ringelement (26) ein geschlitzter Ring ist und/oder dass das Ringelement (26) radial elastisch komprimierbar ist.

5. Implantierbare Vorrichtung nach zumindest einem der vorstehenden Ansprüche,
**dadurch gekennzeichnet, dass**
eine radial äußere Geometrie des Ringelements (26) zumindest abschnittsweise konusförmig ausgebildet ist, wobei ein Öffnungswinkel (a) des konusförmigen Abschnitts 1° bis 12°, insbesondere 2° bis 8°, beträgt.

6. Implantierbare Vorrichtung nach zumindest einem der vorstehenden Ansprüche,
**dadurch gekennzeichnet, dass**
eine radial innere Geometrie des Feststellelements (16) zumindest abschnittsweise konusförmig ausgebildet ist, wobei ein Öffnungswinkel (a) des konusförmigen Abschnitts 1° bis 12°, insbesondere 2° bis 8°, beträgt.

7. Implantierbare Vorrichtung nach Anspruch 5 und 6,
**dadurch gekennzeichnet, dass**
die beiden konusförmigen Abschnitte komplementär zueinander ausgebildet sind.

8. Implantierbare Vorrichtung nach zumindest einem der Ansprüche 5 bis 7,
**dadurch gekennzeichnet, dass**
der jeweilige konusförmig Abschnitt sich entgegen einer Einführrichtung des Verankerungselements (14) verjüngt.

9. Implantierbare Vorrichtung nach zumindest einem der vorstehenden Ansprüche,
**dadurch gekennzeichnet, dass**
eine mit dem Kopf (18) des Verankerungselements (14) zusammenwirkende radiale Innenseite des Ringelements (26) und/oder zumindest ein mit dem Ringelement (26) zusammenwirkender Abschnitt des Kopfs (18) zur Erhöhung einer zwischen dem Kopf (18) und dem Ringelement (26) wirkenden Reibung oberflächenbehandelt, insbesondere aufgerauht ist.

10. Implantierbare Vorrichtung nach zumindest einem der vorstehenden Ansprüche,
**dadurch gekennzeichnet, dass**
das Feststellelement (16) in die Vertiefung (28) der Öffnung (22) des Implantats (12) einschraubbar ist.

11. Implantierbare Vorrichtung nach zumindest einem der vorstehenden Ansprüche,
**dadurch gekennzeichnet, dass**
die Vertiefung (28) der Öffnung (22) eine konkave Auflagefläche für den Kopf (22) des Verankerungselements (14) aufweist und/oder dass der Kopf (22) des Verankerungselements (14) an dem dem Schaft (20) zugewandten Ende ballig ausgeführt ist, derart, dass das Verankerungselement (14) vor einer Fixierung hinsichtlich seiner Winkellage relativ zu dem Implantat (12) variabel positionierbar ist.

12. Implantierbare Vorrichtung nach zumindest einem der vorstehenden Ansprüche,
**dadurch gekennzeichnet, dass**
der komplette Schaft (20) des Verankerungselements (14), jedoch nicht der Kopf (18) des Verankerungselements (14), durch die Öffnung (22) des Implantats (12) hindurchführbar ist, wobei insbesondere die Dimensionen des Kopfes (18) und der Öffnung (22) derart aufeinander abgestimmt sind, dass der Kopf (18) nicht durch die Öffnung (22) hindurchführbar ist.

13. Implantierbare Vorrichtung nach zumindest einem der vorstehenden Ansprüche,
**dadurch gekennzeichnet, dass**
das Feststellelement (16) eine Ringmutter ist.

14. Implantierbare Vorrichtung nach zumindest einem der vorstehenden Ansprüche,
**dadurch gekennzeichnet, dass**
eine oder mehrere Schrägen, die beim Zusammensetzen der Implantierbaren Vorrichtung derart wirken, dass das Ringelement (26) radial komprimiert wird, ausschließlich an dem Ringelement (26) und/oder dem Feststellelement (16) ausgebildet sind.

15. Implantierbare Vorrichtung nach zumindest einem der vorstehenden Ansprüche,
**dadurch gekennzeichnet, dass**
die Vertiefung (28) an einer proximalen Seite des Implantats (12) ausgebildet ist.

16. Implantierbare Vorrichtung nach zumindest einem der vorstehenden Ansprüche,
**dadurch gekennzeichnet, dass**
die eine Einführrichtung des Verankerungselements (14) von einer proximalen Seite zu einer distalen Seite des Implantats (12) gerichtet ist.

17. Implantierbare Vorrichtung nach zumindest einem der vorstehenden Ansprüche,
**dadurch gekennzeichnet, dass**
die implantierbare Vorrichtung eine Knochenfixierungsvorrichtung ist, insbesondere zur operativen Versorgung von Frakturen.

18. Implantierbare Vorrichtung nach zumindest einem der vorstehenden Ansprüche,
**dadurch gekennzeichnet, dass**
das Implantat (12) ein Fixierungselement, insbesondere eine Frakturfixierungsplatte, ist.

19. Implantierbare Vorrichtung nach zumindest einem der vorstehenden Ansprüche,
**dadurch gekennzeichnet, dass**
das Ringelement (26) und/oder das Feststellelement (16) radial von dem Kopf (18) angeordnet ist.

## Claims

1. An implantable apparatus, comprising
- an implant (12), wherein the implant (12) has at least one opening (22);
- at least one anchorage element (14) for fastening the implant (12) to at least one bone, wherein the respective anchorage element (14) comprises a shank (20) and a head (18), and wherein at least a part of the shank (20) can be led through the respective opening (22), while the head (18) can be supported in the respective opening (22); and
- a locking mechanism having a radially deformable ring element (26), wherein the opening (22) of the implant (12) has a recess (28) which has a support surface (24) for the head (22) of the anchorage element (14),
**characterized in that**
the locking mechanism comprises a lock element (16), wherein the ring element (26) can be pressed toward the head (18) of the anchorage element (14) by actuating the lock element (16) to fix the head in the opening (22) of the implant (12), and wherein only the ring element (26) and the lock element (16) cooperate directly on the assembly of the implantable apparatus such that the ring element (26) is radially compressed.

2. An implantable apparatus in accordance with claim 1,
**characterized in that**
the implant (12) and the head (18) of the anchorage element (14) are configured for the polyaxial support of the anchorage element (14) in the respective opening (22) of the implant (12).

3. An implantable apparatus in accordance with claim 1 or claim 2,
**characterized in that**
the ring element (26) is arranged between the lock element (16) and the head (18) of the anchorage element (14) and/or around a spherically designed section of the head (18) of the anchorage element (14) in an assembled state of the implantable apparatus.

4. An implantable apparatus in accordance with at least one of the preceding claims,
**characterized in that**
the ring element (26) is a slit ring; and/or **in that** the ring element (26) is radially elastically compressible.

5. An implantable apparatus in accordance with at least one of the preceding claims,
**characterized in that**
a radially external geometry of the ring element (26) is configured conically at least sectionally, with an opening angle (a) of the conical section amounting to 1° to 12°, in particular to 2° to 8°.

6. An implantable apparatus in accordance with at least one of the preceding claims,
**characterized in that**
a radially internal geometry of the lock element (16) is configured conically at least sectionally, with an opening angle (a) of the conical section amounting to 1° to 12°, in particular to 2° to 8°.

7. An implantable apparatus in accordance with claim 5 and claim 6,
**characterized in that**
the two conical sections are configured complementary to one another.

8. An implantable apparatus in accordance with at least one of the claims 5 to 7,
**characterized in that**
the respective conical section tapers against an introduction direction of the anchorage element (14).

9. An implantable apparatus in accordance with at least one of the preceding claims,
**characterized in that**
a radial inner side of the ring element (26) cooperating with the head (18) of the anchorage element (14) and/or at least one section of the head (18) cooperating with the ring element (26) is/are surface treated, in particular roughened, in order to increase a friction acting between the head (18) and the ring element (26).

10. An implantable apparatus in accordance with at least one of the preceding claims,
**characterized in that**
the lock element (16) can be screwed into the recess (28) of the opening (22) of the implant (12).

11. An implantable apparatus in accordance with at least one of the preceding claims,
**characterized in that**
the recess (28) of the opening (22) has a concave support surface for the head (22) of the anchorage element (14); and/or **in that** the head (22) of the anchorage element (14) is spherically designed at the end facing the shank (20) such that the anchorage element (14) can be variably positioned with respect to its angular position relative to the implant (12) before a fixing.

12. An implantable apparatus in accordance with at least one of the preceding claims,
**characterized in that**
the complete shank (20) of the anchorage element (14), but not the head (18) of the anchorage element (14), can be led through the opening (22) of the implant (12), with in particular the dimensions of the head (18) and of the opening (22) being coordinated with one another such that the head (18) cannot be led through the opening (22).

13. An implantable apparatus in accordance with at least one of the preceding claims,
**characterized in that**
the lock element (16) is a ring nut.

14. An implantable apparatus in accordance with at least one of the preceding claims,
**characterized in that**
one or more slopes, which act during the assembly of the implantable apparatus such that the ring element (26) is radially compressed, are only formed at the ring element (26) and/or at the lock element (16).

15. An implantable apparatus in accordance with at least one of the preceding claims,
**characterized in that**
the recess (28) is formed at a proximal side of the implant (12).

16. An implantable apparatus in accordance with at least one of the preceding claims,
**characterized in that**
the one introduction direction of the anchorage element (14) is directed from a proximal side to a distal side of the implant (12).

17. An implantable apparatus in accordance with at least one of the preceding claims,
**characterized in that**
the implantable apparatus is a bone fixing apparatus, in particular for the surgical care of fractures.

18. An implantable apparatus in accordance with at least one of the preceding claims,
**characterized in that**
the implant (12) is a fixing element, in particular a fracture fixing plate.

19. An implantable apparatus in accordance with at least one of the preceding claims,
**characterized in that**
the ring element (26) and/or the lock element (16) is/are arranged radially from the head (18).

## Revendications

1. Dispositif implantable, incluant
- un implant (12), dans lequel l'implant (12) comporte au moins une ouverture (22),
- au moins un élément d'ancrage (14) pour la fixation de l'implant (12) sur au moins un os, dans lequel l'élément d'ancrage respectif (14) inclut une tige (20) et une tête (18), et au moins une partie de la tige (20) est susceptible d'être passée à travers l'ouverture respective (22), tandis que la tête (18) vient se loger dans l'ouverture respective (22), et
- un mécanisme d'arrêt avec un élément annulaire (26) radialement déformable, dans lequel l'ouverture (22) de l'implant (12) comporte un renfoncement (28) qui présente une surface d'appui (24) pour la tête (22) de l'élément d'ancrage (14),
**caractérisé en ce que**
le mécanisme d'arrêt inclut un élément d'immobilisation (16), dans lequel l'élément annulaire (26) est susceptible d'être pressé par actionnement de l'élément d'immobilisation (16) contre la tête (18) de l'élément d'ancrage (14) de fixer celle-ci dans l'ouverture (22) de l'implant (12), et dans lequel dans l'assemblage du dispositif implanté exclusivement l'élément annulaire (26) et l'élément d'immobilisation (16) coopèrent directement de telle façon que l'élément annulaire (26) est comprimé radialement.

2. Dispositif implantable selon la revendication 1,
**caractérisé en ce que** l'implant (12) et la tête (18) de l'élément d'ancrage (14) sont réalisés pour un montage polyaxial de l'élément d'ancrage (14) dans l'ouverture respective (22) de l'implant (12).

3. Dispositif implantable selon la revendication 1 ou 2,
**caractérisé en ce que** l'élément annulaire (26) est agencé, dans la situation assemblée du dispositif implantable, entre l'élément d'immobilisation (16) et la tête (18) de l'élément d'ancrage (14) et/ou tout autour d'une portion, réalisée en forme de bombement, de la tête (18) de l'élément d'ancrage (14).

4. Dispositif implantable selon l'une au moins des revendications précédentes,
**caractérisé en ce que** l'élément annulaire (26) est une bague fendue et/ou **en ce que** l'élément annulaire (26) est susceptible d'être comprimé élastiquement en sens radial.

5. Dispositif implantable selon l'une au moins des revendications précédentes,
**caractérisé en ce qu'**une géométrie radiale extérieure de l'élément annulaire (26) est réalisée au moins par portion en forme de cône, de sorte qu'un angle d'ouverture (a) de la portion conique est de 1° à 12°, en particulier de 2° à 8°.

6. Dispositif implantable selon l'une au moins des revendications précédentes,
**caractérisé en ce qu'**une géométrie radiale intérieure de l'élément d'immobilisation (16) est réalisée au moins par portion en forme de cône, de sorte qu'un angle d'ouverture (a) de la portion conique est de 1° à 12°, en particulier de 2° à 8°.

7. Dispositif implantable selon la revendication 5 et 6,
**caractérisé en ce que** les deux portions coniques sont réalisées complémentaires l'une de l'autre.

8. Dispositif implantable selon l'une au moins des revendications 5 à 7,
**caractérisé en ce que** la portion conique respective va en se rétrécissant en sens contraire à une direction d'introduction de l'élément d'ancrage (14).

9. Dispositif implantable selon l'une au moins des revendications précédentes,
**caractérisé en ce qu'**une face intérieure radiale, coopérant avec la tête (18) de l'élément d'ancrage (14), de l'élément annulaire (26), et/ou au moins une portion, coopérant avec l'élément annulaire (26) de la tête (18), est traitée en surface, en particulier rendue rugueuse, pour augmenter une friction agissant entre la tête (18) et l'élément annulaire (26).

10. Dispositif implantable selon l'une au moins des revendications précédentes,
**caractérisé en ce que** l'élément d'immobilisation (16) est susceptible d'être vissé dans le renfoncement (28) de l'ouverture (22) de l'implant (12).

11. Dispositif implantable selon l'une au moins des revendications précédentes,
**caractérisé en ce que** le renfoncement (28) de l'ouverture (22) comporte une surface d'appui concave pour la tête (22) de l'élément d'ancrage (14), et/ou **en ce que** la tête (22) de l'élément d'ancrage (14) est réalisée sous forme bombée à l'extrémité tournée vers la tige (20) de telle façon que l'élément d'ancrage (14) est susceptible d'être positionné de façon variable, avant une fixation, pour ce qui concerne sa position angulaire par rapport à l'implant (12).

12. Dispositif implantable selon l'une au moins des revendications précédentes,
**caractérisé en ce que** la tige complète (20) de l'élément d'ancrage (14), mais pas la tête (18) de l'élément d'ancrage (14), est susceptible d'être passée à travers l'ouverture (22) de l'implant (12), et en particulier les dimensions de la tête (18) et de l'ouverture (22) sont accordés les unes aux autres de telle façon que la tête (18) ne peut pas être passée à travers l'ouverture (22).

13. Dispositif implantable selon l'une au moins des revendications précédentes,
**caractérisé en ce que** l'élément d'immobilisation (16) est un écrou annulaire.

14. Dispositif implantable selon l'une au moins des revendications précédentes,
**caractérisé en ce qu'**une ou plusieurs pentes, qui coopèrent lors de l'assemblage du dispositif implantable de telle manière que l'élément annulaire (26) est comprimé radialement, sont réalisées exclusivement sur l'élément annulaire (26) et/ou sur l'élément d'immobilisation (16).

15. Dispositif implantable selon l'une au moins des revendications précédentes,
**caractérisé en ce que** le renfoncement (28) est réalisé sur un côté proximal de l'implant (12).

16. Dispositif implantable selon l'une au moins des revendications précédentes,
**caractérisé en ce qu'**une direction d'introduction de l'élément d'ancrage (14) est dirigée depuis un côté proximal vers un côté distal de l'implant (12).

17. Dispositif implantable selon l'une au moins des revendications précédentes,
**caractérisé en ce que** le dispositif implantable est un dispositif de fixation pour os, en particulier pour le traitement opératoire de fractures.

18. Dispositif implantable selon l'une au moins des revendications précédentes,
**caractérisé en ce que** l'implant (12) est un élément de fixation, en particulier une plaque de fixation pour fractures.

19. Dispositif implantable selon l'une au moins des revendications précédentes,
**caractérisé en ce que** l'élément annulaire (26) et/ou l'élément d'immobilisation (16) est agencé radialement depuis la tête (18).
